# EUROPEAN PATENT APPLICATION

(11) **EP 1 743 936 A1**
(43) Date of publication of application: **17.01.2007**
(21) Application number: 05737053.8
(22) Date of filing: 26.04.2005
(51) Int. Cl.: C12N 5/06, C12N 5/10

(54) **MESENCHYMAL STEM CELL PRECURSOR**

(30) Priority: 26.04.2004 JP 2004129863
(71) Applicant: Riken, Wako-shi, Saitama 351-0198 (JP)
(72) Inventor: NISHIKAWA, Shin-I., Ctr for Develop. Biology RIKEN, Kobe-shi, Hyogo 6500047 (JP); ERA, Takumi, Ctr. for Developmental Biology RIKEN, Kobe-shi, Hyogo 6500047 (JP)
(74) Representative: Schlich, George William
(86) International application number: PCT/JP2005/007923
(87) International publication number: WO 2005/108557

(57) **Abstract**

A premesenchymal stem cell differentiated from a pluripotent stem cell in vitro which is positive for Sox1, and a method for the preparation of the same.

## Description

### Technical Field

The present invention relates to premesenchymal stem cells differentiated in vitro from pluripotent stem cells. The present invention also relates to new purification and preparation methods enabling the enrichment of such cells.

### Background Art

Mesenchymal cells, as well as mesodermal cells, are those which have pluripotency allowing the differentiation into osteoblasts, chondrocytes, myoblasts, and adipocytes. While mesodermal cells possessing pluripotent and self-replicative abilities lose their abilities with the progress of development, mesenchymal cells are known to exist as stem cells for a long period within an adult body. Therefore, mesenchymal stem cells are expected to be useful for cell transplantation therapies (Non-Patent Document 1).

Embryonic stem cells (ES cells) are those possessing differentiation pluripotency which are present in an early embryo, and are capable of differentiating into different cells, including germ cells, when injected into another blastocyst. Mouse embryonic stem cells, on which research has most advanced, are cells possessing pluripotent and self-replicative abilities which are established from the inner cell mass within a blastula at day 3.5 of the development. Theses cells can allow their proliferation to be maintained while retaining their undifferentiated states, by means of adding simply serum and a growth factor, referred to as leukemia inhibitory factor (LIF), to usual culture media. A mouse embryonic stem cell is able to differentiate again into all tissue cells in vivo by injecting it into a blastula at day 3.5 of the development and returning the blastula into a foster mother, and thus is utilized for the generation of chimera mice and knocked-out mice. In recent years, it is also possible to manipulate embryonic stem cells in vitro to differentiate them into various cells of mature tissues.
From the differentiation pluripotency and the capability of easy manipulation of embryonic stem cells, as described above, it is expected that they are used, in medical treatments of the future, as a material for transplantation therapies employing cells.

Studies by the present inventors and other groups have shown the appearance of mature cells, when embryonic stem cells are subjected to in vitro forced differentiation (see, for example, Non-Patent Documents 1 to 3). At present, embryonic stem cells are believed to reach fully mature cells via stem cells of various differentiation stages, although the process of their differentiation is still unknown in many aspects. With respect to the differentiation from embryonic stem cells into mesenchymal cells, there are known methods for the differentiation into adipocytes, chondrocytes, and osteoblasts (see, for example, Non-Patent Document 5), but it is not clear through what process they differentiate into these cells.

[Non-Patent Document 1]
   Mark F. Pittenger and nine others, Science (USA), 1999, No. 284, pp. 143-147
[Non-Patent Document 2]
   Shinichi NISHIKAWA and four others, Development (UK), 1998, No. 125, pp. 1747-1757
[Non-Patent Document 3]
   Toru NAKANO and two others, Science (USA), 1994, No. 265, pp. 1098-1101
[Non-Patent Document 4]
   Takumi ERA and four others, Blood (USA), 2000, No. 95, pp. 870-878
[Non-Patent Document 5]
   Norio NAKATSUJI (ed.), Experimental Courses in the Post-Genome Era 4: Research Protocols for Stem Cells and Clones, in Zikken Igaku (Experimental Medicine) Suppl., Yodo-sha (2001)

### Disclosure of the Invention

### Problems to be Solved by the Invention

A technical object of the present invention is to obtain a premesenchymal stem cell differentiated in vitro from a pluripotent stem cell. Said premesenchymal stem cell differentiates into mesenchymal stem cells.

### [Means for Solving the Problems]

In order to achieve the above-described object, the present inventors have conducted extensive research, with the result that it has been found that when embryonic stem cells are cultured in the presence of retinoic acid, premesenchymal stem cells appear at early stages of differentiation and surprisingly the population of these cells expresses a neuroectodermal marker, Sox1. Based on these findings, the present inventors have reached the completion of the present invention.

The present invention provides a premesenchymal stem cell differentiated in vitro from a pluripotent stem cell. This cell is positive for Sox1 and possesses the ability of differentiation into mesenchymal stem cells.

The present invention provides a method for the preparation of a premesenchymal stem cell, comprising the steps of:
a) culturing and differentiating a pluripotent stem cell; and
b) selecting and separating a cell expressing Sox1.
   The present invention also provides a premesenchymal stem cell obtainable by this method.

### Effect of the Invention

The present invention makes it possible to purify to a high degree and prepare a premesenchymal stem cell differentiated in vitro from a pluripotent stem cell. The premesenchymal stem cell thus obtained will differentiate into mesenchymal stem cells, which are able to differentiate into mesenchymal cells, such as adipocytes, osteoblasts, and others. The cell of the present invention, therefore, is highly versatile as a cell for research and development, for example, of drugs, and is useful in cell transplantation therapies. The present invention is also useful for improving efficiency in differentiating a pluripotent stem cell in vitro to obtain mesenchymal cells of interest.

### Brief Description of the Drawings

Fig. 1 represents the results of RT-PCR showing the expression pattern of ectodermal, mesodermal, mesendodermal, and endodermal markers for embryonic stem cells whose differentiation was induced with and without the addition of retinoic acid.
Fig. 2 represents the results of FACS showing the change in the amount of expression of Sox1-GFP.
Fig. 3 represents the results in which Sox1-GFP-positive and negative cells were purified employing a flow cytometer and analysis was made as to which fraction lead to the appearance of mesenchymal stem cells capable of the differentiation into adipocytes.
Fig. 4 represents the results in which analysis was made, employing a flow cytometer, as to whether or not PDGFRα-positive cells differentiated from among the Sox1-GFP-positive cells and differentiated into adipocytes.

### Best Mode for Carrying Out the Invention

The present invention provides a premesenchymal stem cell differentiated in vitro from a pluripotent stem cell, wherein the cell is positive for Sox1. Preferably, the pluripotent stem cell is derived from a mammal. Additionally and preferably, the pluripotent stem cell is an embryonic stem cell. In the present invention, Sox1 may have a labeling protein fused thereto. The labeling protein is preferably green fluorescent protein (GFP). The premesenchymal stem cell of the present invention can be used for obtaining a mesenchymal stem cell.

The present invention also provides a method for the preparation of a premesenchymal stem cell, comprising the steps of:
a) culturing and differentiating a pluripotent stem cell; and
b) selecting and separating a cell expressing Sox1.

In the method of the present invention, the pluripotent stem cell is preferably derived from a mammal. Additionally and preferably, the pluripotent stem cell is an embryonic stem cell. In the method of the present invention, Sox1 may have a labeling protein fused thereto. The labeling protein is preferably green fluorescent protein (GFP).

In step a) of the present invention, the pluripotent stem cell may be cultured on a culture plate coated with collagen IV. In addition, retinoic acid may be added to the medium in step a), and its concentration is preferably about 10⁻⁷ M.

It is preferable that step b) is performed at 4 days after step a) is started. Step b) may be carried out by a FACS.

The premesenchymal stem cell obtainable by the preparation method of the present invention is also included in the present invention and can be used for obtaining a mesenchymal stem cell.

The present invention also provides a method for the preparation of a mesenchymal stem cell, comprising the steps of:
a) culturing the cell described above or the cell obtained by the methods described above;
b) identifying the appearance of a cell having a stroma cell-like morphology; and
c) selecting and separating a cell which is PDGFRα-positive and FLK1-negative.
   The mesenchymal stem cell obtainable by this method is also within the present invention.

Alternatively, there is provided a method for the preparation of a mesenchymal stem cell, comprising the steps of:
a) culturing and differentiating a pluripotent stem cell;
b) selecting and separating a cell expressing Sox1;
c) culturing the cell separated in b) and identifying the appearance of a cell having a stroma cell-like morphology; and
d) selecting and separating a cell which is PDGFRα-positive and FLK1-negative.
   The mesenchymal stem cell obtainable by this method is also within the present invention.

As used herein, a "mesenchymal cell" means a cell forming a mesenchymal tissue, such as osteoblat, chondrocyte, myoblast, adipocyte, stroma cell, tendon cell, and the like, a mesenchymal stem cell capable of differentiating into these cells, and its premesenchymal stem cell. Mesenchymal cells generated during the embryo development, mesenchymal cells within an animal body, and mesenchymal cells differentiated and generated from pluripotent stem cells in vitro or in vivo are all encompassed in the term "mesenchymal cell."

As used herein, a "mesenchymal stem cell" means a mesenchymal cell possessing the ability of differentiating into mesenchymal cells of one or more types and the ability of self-replication. The mesenchymal stem cell differentiated from a pluripotent stem cell in vitro is positive for PDGFRα and negative for FLK1. Mesenchymal stem cells are able to differentiate into osteoblasts, chondrocytes, myoblasts, adipocytes, stroma cells, tendon cells, and the like, as with mesodermal cells.

As used herein, a "premesenchymal stem cell" means a mesenchymal cell possessing the ability of differentiating into mesenchymal stem cells of one or more types and the ability of self-replication. The premesenchymal stem cell differentiated from a pluripotent stem cell in vitro expresses Sox1, a neuroectodermal marker. The premesenchymal stem cell is able to differentiate into a mesenchymal stem cell which is PDGFRα-positive and FLK1-negative.

The present inventors previously found that when embryonic stem cells were subjected to in vitro differentiation, mesenchymal stem cells appeared at early stages of development and expressed two types of particular cell-surface markers, namely, platelet derived growth factor (PDGFRα) and fetal liver kinase 1 (FLK1), in a particular manner (see, WO2004/106502). Based on this finding, the present inventors have developed for the first time the method for preparing from a pluripotent stem cell differentiated in vitro a mesenchymal stem cell which is at an early developmental stage and such a cell. This method is
characterized by comprising the steps of a) culturing and differentiating a pluripotent stem cell; b) identifying the appearance of a cell having a stroma cell-like morphology; and c) selecting and separating a cell which is PDGFRα-positive and FLK1-negative. The mesenchymal stem cell thus prepared is able to differentiate into mesenchymal cells, such as adipocytes, osteoblasts, chondrocytes, and the like. These cells are highly versatile as cells for research and development, for example, of drugs. For example, adipocytes are useful for developing drugs for the treatment of diabetes and hyperlipemia. In addition, these cells can be used as cells producing hormones, physiologically active substances, and the like, and are also applicable to plastic surgery and cell transplantation therapies, such as mammoplasty employing adipocytes.

The present invention provides a premesenchymal stem cell which is at an earlier developmental stage than the mesenchymal cell of the above-described invention. Since this cell differentiates into a mesenchymal stem cell which is PDGFRα-positive and FLK1-negative as described above, the yield of the mesenchymal stem cell will be improved when the method of the above-described inventions is carried out using the premesenchymal stem cell of the present invention, instead of a pluripotent stem cell. In the case where a pluripotent stem cell is cultured to obtain a mesenchymal stem cell, the yield of the mesenchymal stem cell will be improved, also by carrying out the step of separating a Sox1-expressing cell prior to the step of cell selection according to the expression manner of PDGFRα and FLK1.

As used herein, "in vitro" means that reactions and culturing are carried out outside a living body, including an embryo. In in vitro culturing and/or differentiating of cells, any media, reagents, and containers suitable for cell growth can be used. "In vivo" as used herein means that reactions and culturing are carried out inside a living body, including an embryo, or that a certain phenomenon takes place inside a living body.

As used herein, a "pluripotent stem cell" means a stem cell capable of self-replication which has the ability of differentiating into stem cells of at least two types selected from ectodermal, mesodermal, and endodermal stem cells and includes an embryonic stem cell, an embryonic germ cell (EG cell), an embryonal carcinoma cell (EC cell), a multipotent adult progenitor cell (MAP cell), an adult pluripotent stem cell (APS cell), a bone-marrow stem cell, and the like. Pluripotent stem cells can be used which are derived from various animals, such as mammals, including humans, simians, mice, rats, hamsters, rabbits, guinea pigs, cattle, pigs, dogs, horses, cats, goats, and sheep, birds, and reptiles. Pluripotent stem cells are usually derived from mammals.

As used herein, an "embryonic stem cell" means a cell having differentiation pluripotency which is present in an early embryo, wherein when injected into another blastocyst, the cell is able to differentiate into various types of cells, including germ cells. In the present invention, an embryonic stem cell which has been established newly from the inner cell mass within a blastula may be used, or alternatively a cell line which has been already established may be used.

Sox1 is a transcription factor of the SOX family and is
characterized by having a DNA binding motif termed HMG box. The HMG box was originally found as a motif related to Sry, a sex-determining transcription factor. The Sox family is widely involved in the differentiation in mammals, for example, sex determination, differentiation of nerves, blood cells, blood vessels, and others. SOX1 is not only important in neural differentiation, but also plays important roles in the development of lens in an eye (see, Pevny, L.H., Sockanathan, S., Placzek, M. et al., "A role for Sox1 in neural determination", Development, 125:1967-1978, 1998)

PDGFRα is a transmembrane receptor and has tyrosine kinase activity in its intracellular segment (Soriano, P., Development, 124:2691-2700, 1997). Its ligand is platelet derived growth factor. PDGFRα-deficient mice cause disorders in the formation and differentiation of the somite and blood vessels.

FLK1, similarly to PDGFRα, is a transmembrane receptor and has tyrosine kinase activity in its intracellular segment (Shalaby, F., Cell, 89:981-990, 1997). Its ligand is vascular endothelial growth factor (VEGF). FLK1-deficient mice cause disorders in the differentiation of blood and vascular endothelial cells and are of fetal lethality.

As used herein, "positive" for a particular molecule means that the molecule is expressed by a cell, and "negative" means that the molecule is not expressed. One can determine whether or not a cell expresses a particular molecule, for example, by a FACS, as is described below.

In order to maintain pluripotent stem cells at their undifferentiated states, a maintaining medium for pluripotent stem cells is used. For example, a maintaining medium for pluripotent stem cells is usually a medium in which a minimal medium for culturing cells is supplemented with serum, LIF, L-glutamine, 2-mercaptoethanol, and the like, and an example of its composition is 85% KNOCKOUT D-MEM, 15% FBS, 10⁻⁴ M 2-ME, 2mM L-glutamine, 0.1 mM NEAA, and 1000 U/ml LIF. For Sox1-knocked-in embryonic stem cells described hereinafter, an example of the composition is 90% Glasgow's minimal essential medium, 10% FBS, 10⁻⁴ M 2-ME, 2mM L-glutamine, 0.1 mM NEAA, and 1000 U/ml LIF.

Specific procedures in the method of culturing pluripotent stem cells according to the present invention can be in accordance with procedures and conditions routinely used in the art. Such procedures can be determined as appropriate, for example, in consideration of the description in Norio NAKATSUJI (ed.), Experimental Courses in the Post-Genome Era 4: Research Protocols for Stem Cells and Clones, in Zikken Igaku (Experimental Medicine) Suppl. Yodo-sha (2001); Hogan, G. et al. (ed.), Manipulating the Mouse Embryo: A Laboratory Manual, Cold Spring Harbor Laboratory Press, Plainview, NY (1994); Robertson, E.J. (ed.), Teratocarcimas and Embryonic Stem Cells: A Practical Approach, IRL Press, Oxford, UK (1987), and others.

Taking embryonic stem cells as an example, typical passage procedures and culture conditions are explained as follows. Accordingly, a dish is coated with gelatin, and seeded with embryonic stem cells at a concentration of 10,000 cells/cm² and cultured in an incubator at 37 °C and 5% CO₂. Next day, the medium is exchanged once. When the cells reach confluence at day 2, the cells are rinsed once or twice with phosphate buffered saline, followed by the addition of a sufficient volume of 0.25% (w/v) trypsin-EDTA to cover the cell layer and leaving it as it is for about five minutes. The trypsin solution is removed, an appropriate volume of a culture medium for embryonic stem cells is added, and the cells are detached from the dish by pipetting. The cell suspension is usually centrifuged to precipitate the cells. After the removal of the supernatant, the precipitated cells are resuspended in the culture medium for embryonic stem cells, and seeded and cultured again on a gelatin-coated dish at a concentration of 10,000 cells/cm².

For the differentiation of pluripotent stem cells, pluripotent stem cells are cultured in a differentiation medium for embryonic stem cells where LIF is omitted from the above-described maintaining medium for embryonic stem cells. An example of its composition is 90% α-MEM, 10% FBS, 5 x 10⁻⁵ M 2-ME, and 2 mM L-glutamine. When pluripotent stem cells are cultured in a differentiation medium for embryonic stem cells, the pluripotent stem cells will come out of their undifferentiated states and start the differentiation into a variety of cells. Days of culturing, as stated herein, mean days when culturing is carried out in a differentiation medium for embryonic stem cells.

In the method of the present invention, treatments can be applied which allow pluripotent stem cells to differentiate into mesenchymal cells in the presence of retinoic acid. For embryonic stem cells, for example, a differentiation medium for embryonic stem cells containing 90% α-MEM, 10% FBS, 5 x 10⁻⁵ M 2-ME, and 2 mM L-glutamine is used to culture embryonic stem cells on a dish coated with collagen IV, and retinoic acid is added to the culture medium at day 2 and day 3 of culturing or from day 2 to day 3 of culturing and incubated.

It is possible to add other substances useful for culturing, such as antibiotics, to culture media and differentiation media for pluripotent stem cells.
Substitutes having equivalent functions may be also used, instead of one or more of the constituents of a medium. Each constituent of a medium is sterilized and used in its suitable way.

A fluorescent activated cell sorter (FACS) can be used when cells are selected taking as an indicator the expression of marker genes, such as Sox1, PDGFRα, FLK1, and others. A FACS is usually equipped with a flow cytometer, a laser generator, an optical system, a date processor, and a cell sorter. The function of a FACS is automatic separating of fluorescently labeled cells and computer analysis of the intensity of fluorescence. A FACS irradiates a laser beam, on the way of the flow channel, to cells which are fluorescently labeled with a specific substance, measures signal information of scattering light (forward and lateral scattering light) and fluorescence light for each cell, and displays the result, for example, as a frequency distribution and allows sorting of cells which bring about a specific signal information. FACS instruments are commercially available, for example, from Becton-Dickinson, and can be operated by those skilled in the art following the manufacturer's instructions.

Use can be made, for example, of methods by which Sox1 is substituted with a labeling protein, or with a fused protein having a labeling protein attached to Sox1, in order to select and separate Sox1 expressing cells with ease. The labeling protein is preferably fluorescent proteins, such as green fluorescent protein (GFP), red fluorescent protein (DsRED), yellow fluorescent protein (YFP), and cyan fluorescent protein (CFP); surface antigens, such as Tac antigen, and others. Alternatively, any proteins which can be utilized in a fluorescent activated cell sorter (FACS) can be used. When the cell of the present invention is to be used for transplantation therapy, it is desirable that the labeling protein is a protein which does not adversely affect the vital activity of a recipient animal. Substituting can be achieved by genetic engineering procedures well known to those skilled in the art, such as homologous recombination, gene introduction, and the like. It is desirable that a gene expressing the substituted protein is inserted into the Sox1 locus by homologous recombination procedures, so that the gene is expressed in the same manner of expression as the endogenous Sox1 gene. These procedures for manipulating genes are well known to those skilled in the art (see, Gene Targeting, a practical approach, edited by A.L. Joyner, Oxford University Press, 2000, pp. 1-99).

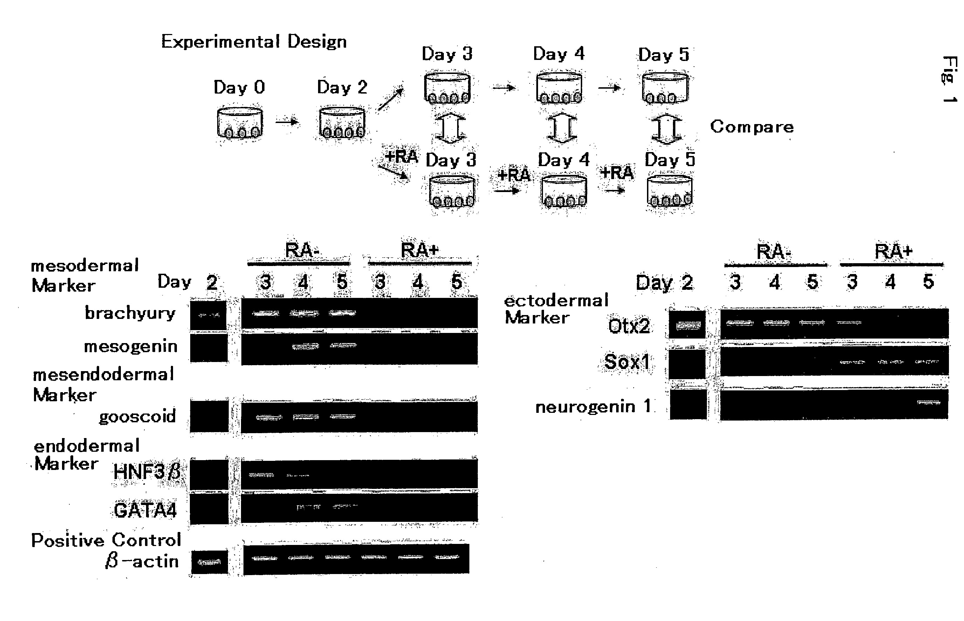

Differentiating of the premesenchymal stem cell of the present invention leads to the appearance of mesenchymal stem cells having a stroma cell-like morphology. Stroma cells have shapes resembling closely to those of fibroblasts, but have larger cytoplasms than those of fibroblasts and slightly rounded shapes, typically shapes as seen in Fig. 1C (left) (see, Kodama, HA et al., J. Cell. Physiol., 1982, 112:89-95). In the method of preparing the mesenchymal stem cell of the present invention, it is possible to select and separate a cell which is PDGFRα-positive and FLK1-negative at the time when cells having a stroma cell-like morphology occupy more than 1%, preferably more than 5%, most preferably more than 10%, of the whole cells. Under usual culture conditions where undifferentiated states are not maintained, the appearance of cells having a stroma cell-like morphology begins around day 5 of culturing. The morphology of cells can be observed under common optical microscopes, phase contrast microscopes, phase contrast inverted microscopes, and the like.

Antibodies for use in the present invention with respect to PDGFRα and FLK1 are polyclonal or monoclonal antibodies, with monoclonal antibodies being preferred for use in a FACS. Such antibodies can be prepared by those skilled in the art with reference to the methods described in the Example, while commercially available antibodies may be used. An anti-PDGFRα monoclonal antibody (Cat. No. 558774) and an anti-FLK1 monoclonal antibody (Cat. No. 555308) are on the market from BD Pharmingen and readily available.

Regarding the method of selecting Sox1-positve cells of the cells in the present invention, the method of using a protein fused with a labeling protein has been described in detail, and it is also possible that selecting is carried out taking the presence of Sox1 mRNA as an indicator. In addition, the method of using an antibody has been detailed about the method of selecting a cell which is PDGFRα-positive and FLK1-negative, and it is also possible that selection is carried out using a fused protein with a labeling protein or taking the presence of mRNAs of these molecules as an indicator.

The present invention will now be described in detail by way of example, which is only illustrative of one embodiment of the present invention and is not intended in any way to limit the present invention thereto.

### Example 1

### Purification of premesenchymal stem cells differentiated from embryonic stem cells

### Materials and methods

### 1. Maintaining embryonic stem cells

### a. Materials

The reagents and equipments listed in Table 1 were used for maintaining embryonic stem cells.

**Table 1**

| Reagent or Equipment | Manufacturer | Cat. No. |
|---|---|---|
| KNOCKOUT Dulbecco's minimal essential medium (D-MEM) | Invitrogen | 10829-018 |
| Glasgow's minimal essential medium (G-MEM) | Invitrogen | 11710-035 |
| Gelatin | SIGMA | G2500 |
| 2-Mercaptoethanol (2-ME) | SIGMA | M7522 |
| Dulbecco's phosphate buffered saline MgCl₂(-), CaCl₂(-) | Invitrogen | 14190-250 |
| L-Glutamine 200 mM | Invitrogen | 25030-081 |
| Non-essential amino acids (NEAA) | Invitrogen | 11140-050 |
| Fetal bovine serum (FBS) | EQUITECH | SFB30-960 |
| LIF | Chemicon | ESG1107 |
| 0.25% (w/v) trypsin-EDTA | Invitrogen | 25200-072 |
| 6 cm dish | FALCON | 35 3802 |

The composition of a medium for maintaining embryonic stem cells was: 85% KNOCKOUT D-MEM, 15% FBS, 10⁻⁴ M 2-ME, 2 mM L-glutamine, 0.1 mM NEAA, and 1000 U/ml LIF. The composition of a medium for maintaining Sox1-knocked-in embryonic stem cells was: 90% G-MEM, 10% FBS, 10⁻⁴ M 2-ME, 2 mM L-glutamine, 0.1 mM NEAA, and 1000 U/ml LIF.

As an embryonic stem cell, a CCE embryonic stem cell derived from mouse 129sv strain was used (Robertson, E. et al., Nature, 323, 445-448, 1986). The Sox1-knocked-in cell was a cell kindly gifted, which was produced by Austin Smith et al. using an embryonic stem cell derived from a mouse 12901a strain, E14tg2a (Aubert J, Stavridis MP, Tweedie S, et al., "Screening for mammalian neural genes via fluorescence-activated cell sorter purification of neural precursors from Sox1-gfp knock-in mice", Proc. Natl. Acad. Sci. USA Suppl 1:11836-11841, 2003).

### b. Methods

A 6 cm dish was coated with gelatin. The dish was seeded with 2 x 10⁵ embryonic stem cells. Next day, the medium was exchanged once. When the cells reached confluence at day 2, the cells were detached from the dish using trypsin and seeded again onto a gelatin-coated dish at a concentration of 2 x 10⁵ cells. Culturing was carried out in an incubator at 37°C and 5% CO₂.

### 2. Differentiation of embryonic stem cells

### a. Materials

The reagents and equipments listed in Table 2 were used for differentiating embryonic stem cells.

**Table 2**

| Reagent or Equipment | Manufacturer | Cat. No. |
|---|---|---|
| Minimal essential medium α-medium | Invitrogen | 12571-063 |
| 2-Mercaptoethanol (2-ME) | SIGMA | M7522 |
| Dulbecco's phosphate buffered saline MgCl₂(-), CaCl₂(-) | Invitrogen | 14190-250 |
| L-Glutamine, 200 mM | Invitrogen | 25030-081 |
| Penicillin-Streptomycin (P/S) | Invitrogen | 15140-122 |
| Fetal bovine serum (FBS) | Invitrogen | US128311 |
| BIOCOAT collagen IV-coated 10 cm dish | Becton-Dickinson | 35 4453 |
| Cell separation buffer | Invitrogen | 13150-016 |
| trans-retinoic acid (RA) | SIGMA | R2625 |
| Insulin | SIGMA | I5523 |
| Dexamethasone | SIGMA | D2915 |
| 3-Isobutyl-1-methylxanthine | SIGMA | 17018 |
| Troglitazone | Sankyo | |

The composition of a differentiation medium for embryonic stem cells was: 90% α-MEM, 10% FBS, 5 x 10⁻⁵ M 2-ME, and 2 mM L-glutamine.

### b. RT-PCR procedures for examining effects of RA on gene expression

A BIOCOAT collagen IV-coated 10 cm dish was seeded with 1 x 10⁵ CCE embryonic stem cells. Culturing was carried out with and without RA having a concentration of 10⁻⁷ M which was added at days 2, 3, and 4 of the induction. The cells of respective treatments were collected at days 3, 4, and 5 to isolate RNA. The isolation of RNA was performed using Trizol (Invitrogen, #15596-026) according to the attached protocol. Then, a cDNA synthesis kit (Invitrogen, #11904-018) was employed according to the attached protocol to synthesize cDNA, and gene expression was examined with routine RT-PCR procedures employing synthetic primers for endodermal, mesodermal, and ectodermal marker genes.

### c. Induction of the differentiation into adipocytes

The induction into adipocytes was carried out by adding the reagents listed in Table 3 to the differentiation medium for embryonic stem cells which did not contain 2-ME. The cells were cultured on a collagen IV-coated dish and the medium was exchanged once every third day.

**Table 3**

| Reagent | Final Concentration |
|---|---|
| Insulin | 5 µg/ml |
| Dexamethasone | 1 µM |
| 3-Isobutyl-1-methylxanthine (IBMX) | 500 µM |
| Troglitazone | 1 µM |

### 3. Preparation of antibodies

A monoclonal antibody which recognized an extracellular segment of each molecule was prepared by procedures well known to those skilled in the art. Specific procedures were as follows. cDNA coding for the extracellular segment of mouse PDGFRα was amplified with PCR and the resulting DNA sequence was ligated to a DNA sequence coding for the Fc region of human IgG1 to form a fused cDNA. The cDNA was introduced into COS1 cells, and the fused protein in the culture supernatant was collected using a Protein A column. The collected protein was subjected to immunization of rats. After the immunization was completed, spleen was removed and spleen cells were fused with myeloma cells (X63.Ag8), to produce hybridoma cells. In order to obtain an antibody of interest, an antibody reacting with Balb/c-3T3 cells which express the fused protein and PDGFRα was selected from among antibodies contained in the hybridoma-cell culture supernatants, and a clone of the hybridoma cell producing the antibody was identified. These procedures yielded monoclonal antibodies which specifically recognized PDGFRα.

### 4. Antibody staining and selection of cells with FACS vantage or FacsAria

### a. Preparation of reagents

The reagents listed in Table 4 were used.

**Table 4**

| Reagent | Manufacturer | Cat. No. |
|---|---|---|
| 10x Hank's balanced salt solution (10x Hank's buffer) | Invitrogen | 14185-052 |
| Bovine serum albumin (BSA) | SIGMA | A-2153 |

To 900 ml of deionized water, 100 ml of 10x Hank's buffer and 10 g of BSA (final concentration, 1%) were added and well stirred. After the BSA was dissolved, the solution was filter sterilized through a 0.2 µm filter.

### b. Method

For the purification of GFP-Sox1-positive cells, the cells were separated in the cell separation buffer and then the cells were dissolved in 1% BSA/Hank's buffer to 10⁶ cells/ml in the case of using FACS Vantage or to 10⁷ cells/ml in the case of using FacsAria and used for cell selection.

For the purification of PDGFRα-positive cells, an anti-PDGFRα antibody was labeled with biotin (Molecular Probe) and used in staining as described below. Cells were separated in the cell separation buffer, and then 10 µl of mouse serum per 10⁶ cells was added and incubated on ice for 20 minutes. Subsequently, 100 ng or 500 ng of the antibody was added and incubated on ice for 20 minutes. After 20 minutes, the cells were washed once with 1% BSA/Hank's buffer. The cells were resuspended in 500 µl of 1% BSA/Hank's buffer containing streptoavidin-allopycocyanin (APC; Becton-Dickinson) and incubated on ice for 20 minutes. Finally, the cells were washed twice with 1% BSA/Hank's buffer and dissolved in 1% BSA/Hank's buffer to 10⁶ cells/ml in the case of using FACS Vantage or to 10⁷ cells/ml in the case of using FacsAria and used for cell selection.

For the selection of FLK1-negative cells, an anti-FLK1 antibody was labeled with phycoerythrin (both of which were purchased from Molecular Probe) and used in staining similarly to the above.

Procedures of using FACS Vantage (Becton-Dickinson) and FacsAria (Becton-Dickinson) were in accordance to the attached guidebook. For FACS Vantage, the frequency of oscillation of the nozzle was on the order of 26000, the level was 3V, and the drop delay was about 12-14.

### Results and discussion

### 1. RA suppresses the expression of mesodermal, mesendodermal, and endodermal markers.

RNA was extracted from the embryonic stem cells whose differentiation was induced with and without the addition of RA and the expression of ectodermal, mesodermal, mesendodermal, and endodermal markers was examined, in order to investigate effects of the addition of RA on in vitro differentiation of embryonic stem cells, in particular, gene expression (Fig. 1). It turned out that by the addition of RA, the expression of the endodermal, mesendodermal, and endodermal markers was strongly suppressed, while the ectodermal markers, in particular, Sox1, a neuroectodermal marker was strongly induced.

### 2. Establishing of the Sox1-GFP-knocked-in embryonic stem cell

Austin Smith et al. prepared the construct in which a green fluorescent protein (GFP) gene was inserted into a mouse Sox1 gene in frame with its start codon ATG, which was introduced into an embryonic stem cell to generate a homologous recombinant clone (Sox1-GFP-knocked-in embryonic stem cell). The cloned cell was introduced into a mouse blastocyst to examine the expression of the GFP gene in the mouse development. The expression of the GFP gene was observed in the neuroectoderm, which expresses Sox1, and the neural tube and eyeballs. Thus, Sox1 can be used mainly as a neuroectodermal marker (Aubert, J. et al., supra). The Sox1-GFP-knocked-in embryonic stem cell was used to analyze whether or not the mesenchymal stem cells induced similarly by the addition of RA were differentiated from Sox1-positive cells, since in the experiments described in the previous section, the addition of RA strongly suppressed the expression of the endodermal, mesendodermal, and endodermal markers, and induced the expression of Sox1.

### 3. Analysis of the cell origin of employing the Sox1-GFP-knocked-in embryonic stem cell

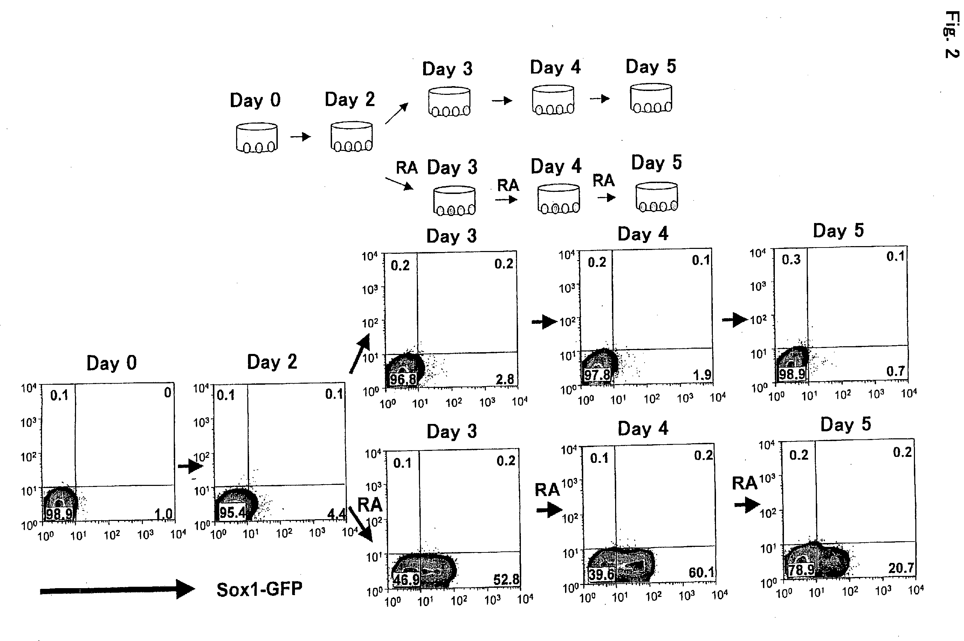

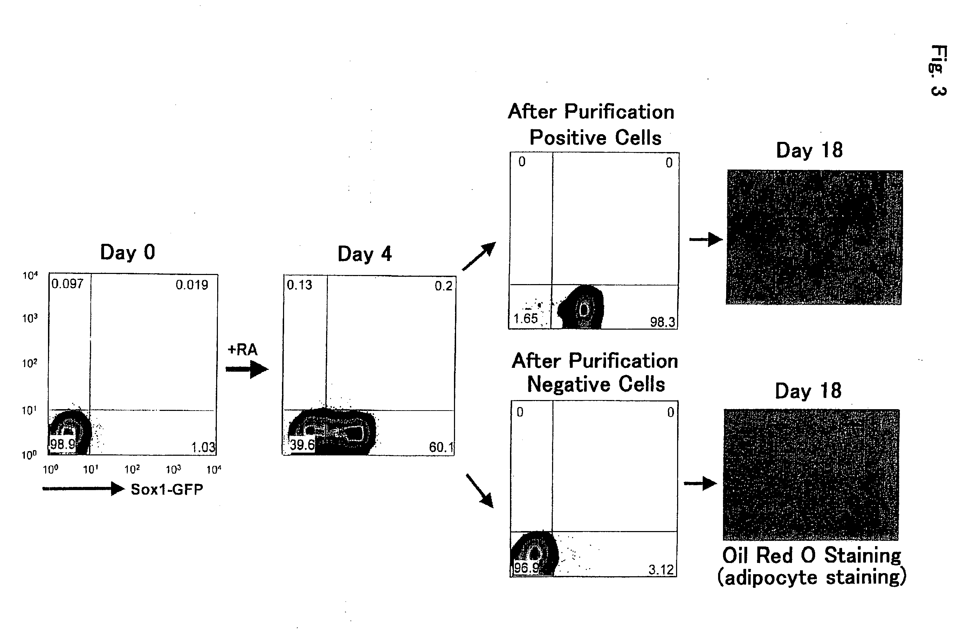

In the culture system established by the present inventors, the Sox1-positive cells reached their maximum at day 4 after the induction of differentiation was begun (Fig. 2). The present inventors previously showed that at this time point, the expression of PDGFRα was not observed yet (see, WO2004/106502). Therefore, Sox1-GFP-positive and Sox1-GFP-negative cells were purified employing a flow cytometer to analyze which fraction gave rise to the appearance of embryonic stem cells capable of differentiating into adipocytes (Fig. 3). The Sox1-GFP-negative cells led to little or no appearance of adipocytes, whereas the culture in which the differentiation was induced from the Sox1-positive cells resulted in observing a plurality of adipocytes (Fig. 3) .

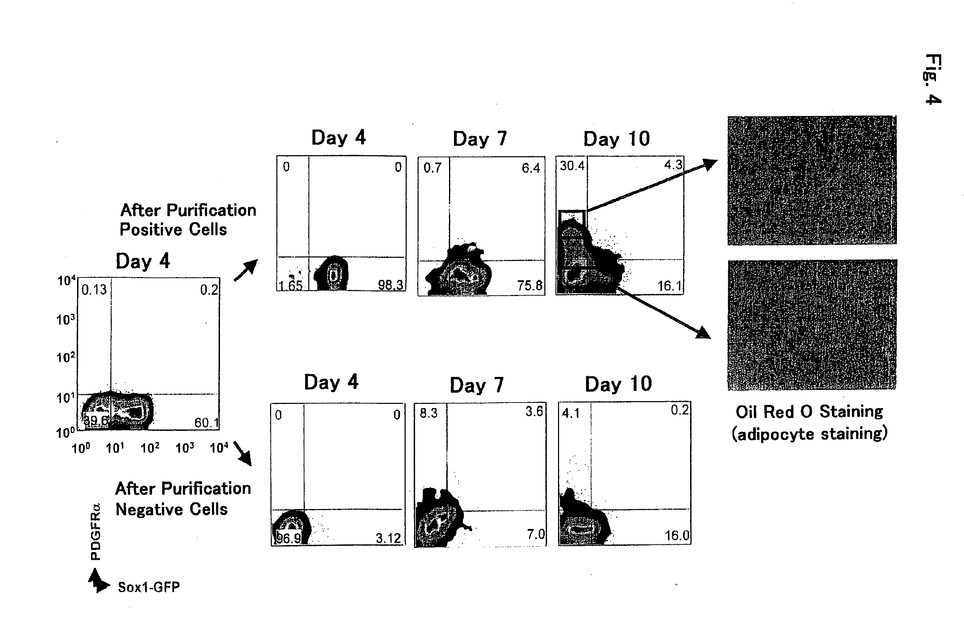

Subsequently, analysis was made as to whether or not the PDGFRα-positive mesenchymal stem cells, which were previously separated by the present inventors (see, WO2004/106502), were differentiated and the differentiated cells were differentiated into adipocytes (Fig. 4). The expression of PDGFRα was analyzed by separating the cells taking the expression of Sox1-GFP as an indicator, followed by subsequent culturing of Sox1-GFP-positive and Sox1-GFP-negative cells. From the Sox1-GFP-positive cells, 30% of the cells were positive for PDGFRα at day 10 after starting the induction of differentiation of the Sox1-GFP knocked-in embryonic stem cell, while the Sox1-GFP-negative cells resulted in the appearance of a small number of PDGFRα-positive cells being observed. The PDGFRα-positive and PDGFRα-negative cells were purified again by a flow cytometer, respectively, and compared for the ability of differentiating into adipocytes. The results revealed that only the PDGFRα-positive cells differentiated from the Sox1-GFP-positive cells differentiated into adipocytes. Taking these results together, it is suggested that the mesenchymal stem cell which was previously separated by the present inventors is differentiated from the cell which expresses Sox1, a neuroectodermal marker.

### Industrial Applicability

The present invention can be applied for research and development, for example, of drugs, and widely utilized in the fields of cell engineering, cell biological studies, and others.

## Claims

1. A premesenchymal stem cell differentiated from a pluripotent stem cell in vitro, wherein the cell is positive for Sox1.

2. The cell according to claim 1, wherein the pluripotent stem cell is derived from a mammal.

3. The cell according to claim 1 or 2, wherein the pluripotent stem cell is embryonic stem cell.

4. The cell according to any of claims 1 to 3, wherein the Sox1 is entirely or partially substituted with a labeling protein.

5. The cell according to claim 4, wherein the labeling protein is green fluorescent protein (GFP).

6. The cell according to any of claims 1 to 5, which is used for obtaining mesenchymal stem cells.

7. A method for the preparation of a premesenchymal stem cell, comprising the steps of:
a) culturing and differentiating a pluripotent stem cell; and
b) selecting and separating a cell expressing Sox1.

8. The method according to claim 7, wherein the pluripotent cell is derived from a mammal.

9. The method according to claim 7 or 8, wherein the pluripotent stem cell is embryonic stem cell.

10. The method according to any of claims 7 to 9, wherein in step a), the pluripotent stem cell is cultured on a culture plate coated with collagen IV.

11. The method according to any of claims 7 to 10, wherein in step a), the pluripotent stem cell is cultured in a medium to which retinoic acid is added.

12. The method according to claim 11, wherein the concentration of retinoic acid is 10⁻⁷ M.

13. The method according to any of claims 7 to 12, wherein step b) is performed at 4 days after step a) is started.

14. The method according to any of claims 7 to 13, wherein the Sox1 is entirely or partially substituted with a labeling protein.

15. The method according to claim 14, wherein the labeling protein is green fluorescent protein (GFP).

16. The method according to any of claims 7 to 15, wherein step b) is by a FACS.

17. A premesenchymal stem cell which is obtainable by a preparation method according to any of claims 7 to 16.

18. A method for the preparation of a mesenchymal stem cell, comprising the steps of:
a) culturing a cell according to any of claims 1 to 6 or to claim 17;
b) identifying the appearance of a cell having a stroma cell-like morphology; and
c) selecting and separating a cell which is PDGFRα-positive and FLK1-negative.

19. A method for the preparation of a mesenchymal stem cell, comprising the steps of:
a) culturing and differentiating a pluripotent stem cell;
b) selecting and separating a cell expressing Sox1;
c) culturing the cell separated in b) and identifying the appearance of a cell having a stroma cell-like morphology; and
d) selecting and separating a cell which is PDGFRα-positive and FLK1-negative.

20. A mesenchymal stem cell which is obtainable by a preparation method according to claim 18 or claim 19.
